# EUROPEAN PATENT SPECIFICATION

(11) **EP 2 498 514 B1**
(45) Date of publication and mention of the grant of the patent: **01.01.2014**
(21) Application number: 11157325.9
(22) Date of filing: 08.03.2011
(51) Int. Cl.: H04R 25/00, A61N 1/36, A61N 1/00, H04B 5/00, H04B 7/08

(54) **A hearing device and method of operating a hearing device**
Hörgerät und Verfahren für den Betrieb eines Hörgerätes
Dispositif auditif et procédé de fonctionnement d'un dispositif auditif

(43) Date of publication of application: 12.09.2012
(73) Proprietor: NXP B.V., 5656 AG Eindhoven (NL)
(72) Inventor: Kerselaers, Anthony, Redhill, Surrey RH1 1DL (GB)
(74) Representative: Hardingham, Christopher Mark

(56) References cited:
- EP-A2- 1 879 426
- WO-A1-2008/113053
- US-A1- 2009 052 707
- US-A1- 2009 154 739
- US-A1- 2010 150 383

## Description

### Field

This invention relates to hearing devices. It further relates to methods for operating hearing devices.

### Background

A non-exclusive example of a hearing device to which this invention relates is that of hearing aids, and in particular binaural hearing aids. Conventional hearing aids include a microphone to convert acoustic waves into an electrical signal; electronics to adapt modify the electrical signal, and a speaker to convert the electrical signal into an acoustic signal in close proximity to an ear.

In the case, for example, of binaural hearing aids, that is to say, a hearing device which has an earpiece typically including a speaker, for each ear of a user, it is known to provide communication between the two earpieces. This communication may typically be by means of near-field communication (NFC). In NFC devices, also commonly referred to as magnetic induction devices, the magnetic near-field, which attenuates with the third power of distance, is used. In the transmitter, a coil is used to generate a varying magnetic field, and this magnetic field induces a current in the coil of the receiver.

It is also known to provide external communication, from for instance a remote control device to an earpiece, by means of NFC, as described for example in United States patent publication US4,918,736. It is yet further known to provide a hearing aid with a wirelessly connected remote processor as discussed in United States patent publication US5,721,783.

In a further development, systems have been developed, in which a first communication is established between the earpiece and the remote control based on inductive near field coupling and a second communication is established between the remote controller and further electronic equipment like cellular phone or other by means of electromagnetic radiation. However, there is a constraint on the distance between the remote controller and of the earpiece is due to the rapid attenuation of the near-field strength.

European Patent Application EP1,879,426 discloses a method and apparatus for binaural hearing assistance systems using monaural audio signals. The system includes hearing assistance devices R1 and R2 and relaying of control signals, audio signals or a combination of both between hearing assistance devices is disclosed.

Increasing miniaturisation of earpieces imposes a further constraint on the receiver for electromagnetic radiation. In particular there may be problems regarding the signal quality at the receiver.

### Summary

According to a first aspect of the invention, there is provided a hearing device comprising a first sub-unit and a second sub-unit as defined in claim 1.

The first sub-unit may be for a first ear and the second sub-unit may be for a second ear.

By providing two antennae, one in each sub-unit, receiver antenna diversity is facilitated overcoming, at least to some extent, the geometrical constraints of an individual earpiece.

In embodiments the second near field communication device is configured to transmit the preferred baseband stream, and the first near field communication unit is configured to receive the preferred baseband stream. In other embodiments, the second near field communication device is configured to transmit the second baseband stream, the first near field communication device is configured to receive the second baseband stream, and the first sub-unit is configured to determine a further preferred baseband stream from at least one of the first baseband stream and the second baseband stream. Thus it may be that either one of the sub-units determines a resultant baseband stream from the first and second baseband streams, or each of the sub-units determines a resultant baseband stream (that is to say, the preferred baseband stream and the further preferred baseband stream respectively) from the first and second baseband streams

In embodiments at least one of the first receiver and the second receiver comprises a transceiver. Having a transmitting function available in the transceiver is useful for handshaking and other synchronisation with the further communication device

In embodiments, the preferred baseband stream is a result of a combination of the first baseband stream and the second baseband stream, or a one of the first baseband stream and the second baseband stream.

The preferred baseband stream is determined based on respective first and second received signal strength indicator or on respective first and second audio stream distortion associated with the respective first and second baseband streams. Similarly, in embodiments in which there is a further preferred baseband stream, the further preferred baseband stream may be determined based on respective first and second received signal strength indicator or on respective first and second audio stream distortion associated with the respective first and second baseband streams.

In embodiments the first and second near field communication devices are configured to operate within a bandwidth which is within the range of 3- 16 MHz. In embodiments the first and second receivers are each configured to receive an electromagnetic signal within one of a group of frequency ranges consisting of 400MHz ― 6 GHz and 2.40-2.48 GHz.

According to another aspect of the invention of there is provided a hearing device being one of the group consisting of: a hearing aid; a pair of headphones; a pair of ear-pieces, and a pair of implants.

According to another aspect of the present invention there is provided a method of operating a hearing device as defined in claim 9.

The method may further comprise transmitting the preferred baseband stream by the second near field communication unit, and receiving the preferred baseband stream by the first near field communication unit by. In other embodiments, of the method may further comprise transmitting the second baseband stream by the second near field communication device, is configured to receiving the second baseband stream by the first near field communication device, and determining a further preferred baseband stream from at least one of the first baseband stream and the second baseband stream by the first sub-unit.

In embodiments, determining a preferred baseband stream from at least one of the first baseband stream and the second baseband stream comprises making a determination based on respective first and second received signal strength indicator or on respective first and second audio stream distortion associated with the respective first and second baseband stream.

These and other aspects will be apparent from, and elucidated with reference to, the embodiments described hereinafter.

### Brief description of Drawings

Embodiments of the invention will be described, by way of example only, with reference to the drawings, in which
Figure 1 is a schematic system diagram of an application of a hearing device according to an embodiment of the invention;
Figure 2 shows the outline of an earpiece according to an embodiment of the invention; and
Figures 3, 4 and 5 are hybrid flow-diagrams of methods of implementing antenna diversity; and
Figure 6 shows a schematic layer structure of a hearing device configured for operation according to embodiments of the invention

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments

### Detailed description of embodiments

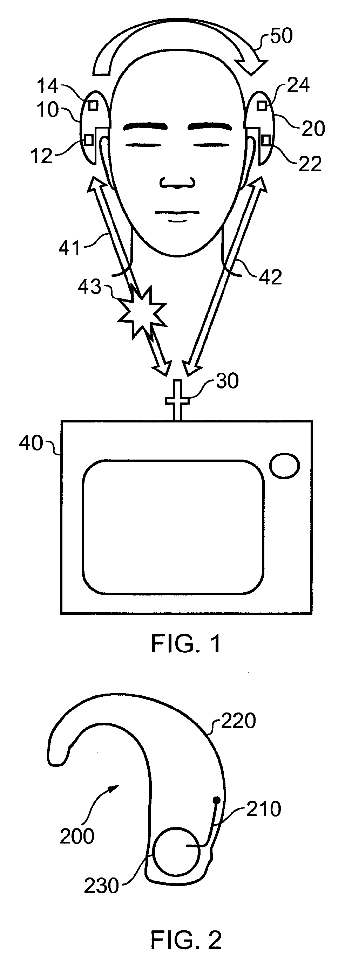

Figure 1 is a schematic system diagram of an arrangement of a hearing device according to an embodiment of the invention. The hearing device comprises two sub-units 10 and 20, which as shown may be individual earpieces, one for each of the left and right ear of the user. It will be immediately appreciated that although the sub-units 10 and 20 are shown as earpieces, they may take a variety of different forms, such as without limitation, ear buds, in-ear hearing aids, headphones, or even may be implanted devices such as Cochlear implants which are widely employed by people with hearing impairments.

Each sub-unit 10, 20 includes a receiver, respectively first receiver 12 and second receiver 22, for receiving a respective signal from an electromagnetic field. The electromagnetic field is generated by an antenna 30 which forms part of a further communication device 40. Further communication device 40 may be, for instance, a television as shown in the figure, or may be part of a two-way communication devices such as a mobile phone.

Further communication device 40 transmits, by means of antenna 30, an electromagnetic signal. Typically, the electromagnetic signal will be transmitted at a frequency of about 2.5 GHz, although the invention is not limited to this frequency range. Non-limiting but preferred frequency ranges for the electromagnetic signal include 400MHz ― 6 GHz, and 2.4 ― 2.48 GHz.

However it is known that for example at a frequency of 2.5 GHz electromagnetic radio signals between devices may face severe propagation fading behaviour. This effect results in reduced signal strength at the receiver input which can decrease below the detectable level and thus interrupt the demodulated output information. For example at 2.5 GHz in a typical indoor situation, regular signal reductions of 10 to 20 db are very realistic.

The effects of propagation fading can be reduced by using antenna diversity. As used herein, the term antenna diversity refers to the use of multiple antennas (also referred to here-in as antennae) so as to enhance the reception of a signal, which in this case is typically an electromagnetic signal.

By using two or more antennas that receive signals which are uncorrelated or are only partly correlated, an improvement can be achieved. However, due to the space constraints usually present within the individual earpieces, it is generally impractical to include more than one antenna in the earpiece.

As shown in figure 1 this problem may be resolved by including an antenna in each of the earpieces. Provided that at least to some extent, the earpieces can operate as part of a single hearing device, that is to say that there is some communication and interaction between the two earpieces, the antenna in the two earpieces may provide suitable antenna diversity so as to mitigate the effects of localised signal reductions. The amount of the improvement depends on the correlation factor between the antennas and the method used for antenna diversity. Examples of the methods used for antenna diversity include selection diversity and maximum ratio combining, each of which will be considered in more detail below. In case of selection diversity the better received signal between those from the two antennae is selected; this allows simple implementations which can be done at the RF stages or in the baseband stages. In case of maximum ratio combining, the two signals are weighted and added together in the correct phase. This method is more complex but delivers more improvement then selection combining.

To provide sufficient benefit from antenna diversity the antenna signals should be sufficiently uncorrelated. Although there is no fixed a lower bound below which no improvement may be demonstrable, a typical good figure of merit is a correlation coefficient of 0.5. To be able to have such uncorrelated antenna signals two or more antennas should be at a certain distance from each other, for example a quarter wavelength.

It will be appreciated by the skilled person from the physical dimensions of a hearing aid earpiece that it is not generally possible, within a single earpiece, to implement two antennas that provide sufficient uncorrelated signals, when operating in radio frequencies which are typically used currently. But, by providing for an antenna in each of the earpieces, one to be worn on either side of the head, the antennae may have a sufficiently large separation to allow them to receive relatively uncorrelated signals from the same source electromagnetic field. The signals received at either antenna are uncorrelated since the antennas have a large separation, the dimension of the head and above all the head itself gives a lot of isolation between the two antennae: the propagation loss between the antennae has been found more than 40dB.

Thus, any local attenuation of interference in the electromagnetic field may produce a deterioration in the received signal at only one of the antennae. Such a situation is shown in figure 1, in which an attenuator 43, which may be for instance a piece of furniture, or a mobbing arm or hand, lies in the first signal path 41 to first sub-unit 10 but does not interrupt the signal path 42 to the other sub-unit 20. A similar situation may apply where there is a strong interference, for instance from an interferer such as a Bluetooth device or a cellular transmitter, which may affect one signal path to a significantly greater level than the other signal path. This will particularly be the case where the interferer is carried by the user, for instance, in a pocket on one side of the user's body.

In order to implement antenna diversity using an antenna on each of the earpieces, communication is necessary between the earpieces. This is shown schematically in figure 1 by communication 50. Although in figure 1 the communication is shown as a one-way communication, the communication will usually be a two-way communication.

Figure 2 shows an outline schematic of a conventional hearing aid 200. The hearing aid includes RF antenna shown at 210. In order to conserve the robustness of the device, the antenna will typically be mounted inside the casing 220. In addition to the RF antenna, the hearing aid includes a loop or coil 230 for NFC.

A variety of ways of implementing antenna diversity according to embodiments of the invention are possible, some of which will now be described.

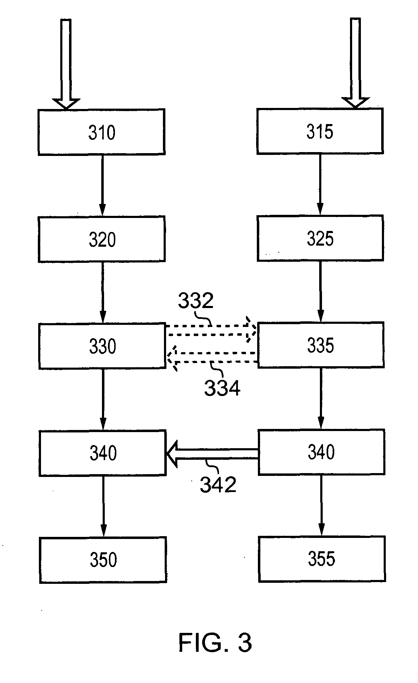

A first method, is depicted in Figure 3, which shows, at the left hand side steps associated with a first sub-unit, and at the right hand side, steps associated with the other sub-unit. At step 310, 315 the first and second receivers 12 and 22 respectively receive an electromagnetic signal, that is to say an RF signal. Each receiver converts the respective RF signal to derive a respective first and second baseband stream, at step 320, 325. Each baseband stream normally contains both the left and right audio channels. It may be encoded in any one of a variety of known coding arrangements, which will be well-known to the skilled person. In addition and also at step 320, 325 each receiver determines the quality of the received RF signal, in the form of a Received Signal Strength Indicator (RSSI).

In this method, at step 330, 335, each sub-unit transmits to the other sub-unit, by means of the NFC link, the RSSI, 332, 334 corresponding to the signal received at its respective receiver. Each sub-unit then determines whether its own RSSI is higher than the other sub-unit's RSSI. The sub-unit that has received the better strength RF signal, that is to say the sub-unit which has the higher RSSI, then transmits, at step 340, its baseband stream 342 to the other sub-units, again by means of the NFC link.

Both sub-units (that is to say both the left ear and right ear sub-units) now have access to the baseband stream derived from the higher RSSI RF signal. Each sub-unit decodes this audio-stream in a conventional manner shown at 350,355, sending the left or right channel to the respective left or right output. That is, each sub-unit directs a single channel to its associated output. Typically the output will be an in-ear or on-ear speaker, although in the case of implants, the skilled person that will appreciate that the output may take the form of electrical stimuli.

Although this method has been described with respect to RSSI, the skilled person will appreciate that in variants of this method, other parameters, such as baseband stream distortion, may be transferred between the sub-units instead of RSSI.

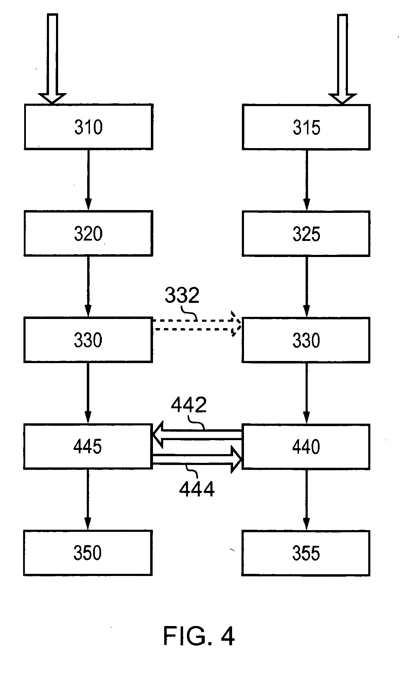

In further variants, such as that shown in Figure 4, the signal quality information such as RSSI is transmitted only from one, sending, sub-unit by the NFC link to the other, receiving, unit. In this figure, the steps corresponding to those in Figure 3 are depicted by the same reference numerals. However, in step 330, RSSI is transmitted (shown at332) only by one sub-unit. The receiving sub-unit compares, at 440, the received information such as RSSI or baseband stream distortion discussed above, with that from its own receiver. If it determines that the signal quality from its own receiver is higher than that of the sending sub-unit, it transmits its own baseband stream to the sending sub-unit by means of the NFC link (shown at 442); otherwise it requests the sending sub-unit to transmit, at 445, its baseband stream, that is to say, the sending sub-unit's baseband stream, to the receiving unit (shown at 444).

Other variants, such as use of a timeout rather than a request for information in order to initiate transfer of the baseband stream, will be immediately apparent to the skilled person.

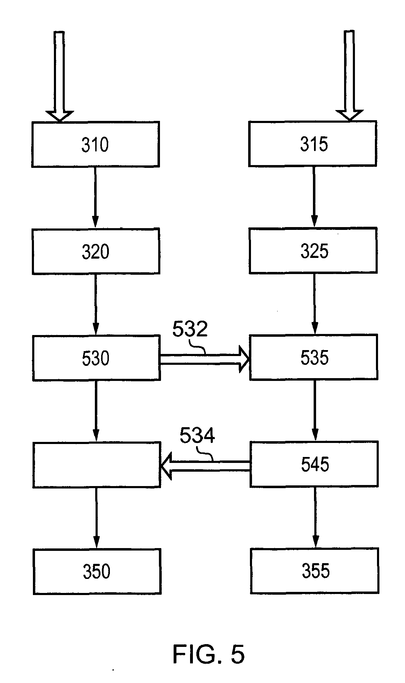

In other embodiments, instead of signal quality information being transferred between the devices, the baseband stream converted by the first sub-unit is transmitted by the NFC link from the first sub-unit to the second sub-unit. This is shown in figure 5, at step 530, at which baseband stream 532 is transmitted from the first to the second sub-unit. In this case, the second sub-unit carries out the determination, at 545, of which baseband stream to use, based on information either associated with the baseband stream itself, or with the RF signal. In the latter case information relating to the RF signal may be also be transmitted from the first sub-unit to the second sub-unit. As a result of the determination made by the second sub-unit, it may require to transmit its own baseband stream (534) to the first sub-unit, in the case that it determines that its own baseband stream is the better quality signal, or, in the case that it determines that to the baseband stream from the first sub-unit is the better quality signal, it may simply transmit confirmation of this to be first sub-unit.

In yet other variants (not shown), each sub-unit transmits its baseband stream to the other sub-unit. Each sub-unit then determines which baseband stream to use, as described above, and outputs the respective channel of that baseband stream to its output.

The embodiments described so far have related to a simple selection of the better quality baseband stream.

In other embodiments, instead of simply selecting the better quality, or stronger, baseband stream, information from the both of the baseband streams is combined in order to provide a resultant preferred baseband stream. It will be apparent that in this case, the term preferred is used to indicate that the resultant baseband stream is preferred over either of the input signals, whilst in embodiments described above, the preferred baseband stream may be an exact copy of one or other of the baseband stream, and thus is the preferred one between the two input signals.

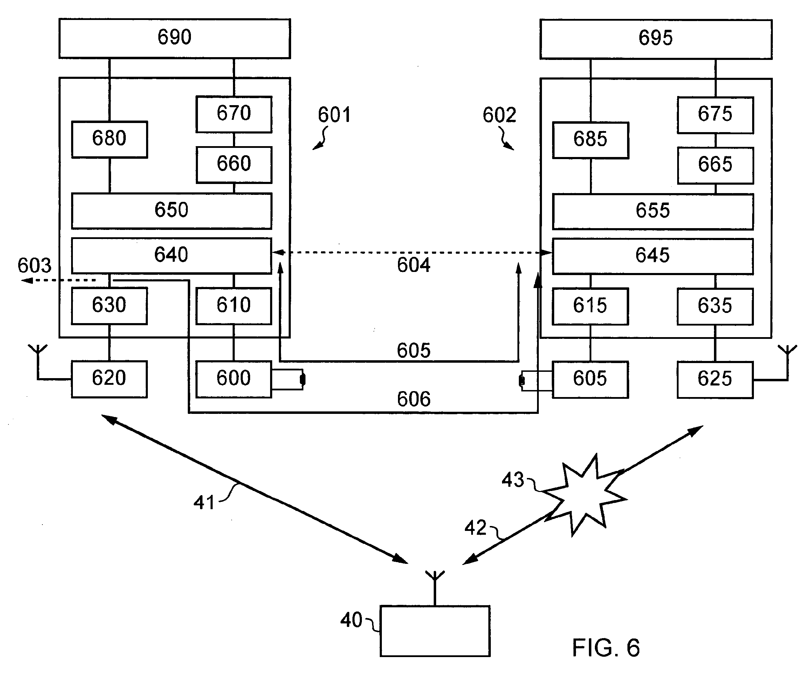

A hearing device, configured for operation according to embodiments of the invention, is shown schematically in Figure 6. The device includes a pair of subunits 601, 602. Each subunit has a magnetic induction radio 600, 605 together with a respective magnetic induction radio interface 610, 615. Each subunit also has a respect to RF radio 620, 625 together with an associated RF radio interface, 630,635.

The magnetic induction radio interface 610,615 and RF radio interface 630, 635 are both connected to a link diversity management MI-RF bridge 640, 645, the pair of bridges together manage the antenna diversity. The link diversity management and MI-RF bridge within each subunit can communicate by dotted link 604. As shown in the route 605, this is effected by means of the respective MI radio interfaces 610, 615, and MI radios 600, 615.

The higher layers comprising the remainder of each subunit 601, 602 are conventional: each subunit includes a new dual PHY MAC and network processor 650, 655. Above this layer is an audio decoder 660, 665 together with an audio interface 670, 675. The processor also controls a data and control interface 680, 685. The audio interface and data and control interface both interface with a host controller sound processor 690, 695.

As described above, each subunit communicates with a further communication device 40 by means of the latter's RF radio and antenna 30. As shown, the signal path 41 to the first sub-unit may result in a better quality signal than that resulting from the second signal path 42 to the second sub-unit, which signal path may include an interruption 43.

The RF signals are received by radio 620, 625, and converted by the RF radio interface 630, 635 in order to produce the respective baseband stream. At least one of link diversity management MI-RF bridge units 640, 645, determines that the baseband signal associated with its subunits should be transferred to the other sub-unit, as shown by routing 606, This transfer is effected by means of the respective MI radio interface 610, MI radio 600, MI radio 605, and MI radio interfaces 615. In the example shown, MI radio 600 acts as transmitter, and MI radio 610 605 acts as receiver for the baseband stream 603. Examples of methods to make the above determination have been described above with respect to figures 3 to 5.

In summary, then, from one viewpoint, a hearing device is disclosed, in which antenna diversity is provided by means of including an antenna in each of two sub-units or earpieces, one being for each ear. The antennae communicate with a further communication device by conventional wireless RF transmission. The two sub-units communicate by means of near field communication, NFC. The two sub-units act in co-operation so as to ensure that a relatively higher quality baseband stream is extracted from either one or a combination of the receivers associated with the antennae. Deleterious effects from e.g. local disturbances to the electromagnetic field of the transmission may thereby be mitigated or overcome. Methods of operating such hearing devices are also disclosed.

For the sake of completeness it is also stated that the term "comprising" does not exclude other elements or steps, the term "a" or "an" does not exclude a plurality, a single processor or other unit may fulfil the functions of several means recited in the claims and reference signs in the claims shall not be construed as limiting the scope of the claims.

## Claims

1. A hearing device comprising a first sub-unit and a second sub-unit:
the first sub-unit (10) comprising a first receiver (12) configured to receive a first signal from an electromagnetic field and derive a first baseband stream therefrom;
the second sub-unit (20) comprising a second receiver (22) configured to receive a second signal from the electromagnetic field and derive a second baseband stream therefrom;
wherein the first sub-unit further comprises a first near field communication device (610) configured to transmit the first baseband stream, the second sub-unit further comprises a second near field communication device (615) configured to receive the first baseband stream, **characterized in that** the second sub-unit is configured to determine a preferred baseband stream from at least one of the first baseband stream and the second baseband stream;
wherein the preferred baseband stream is a result of a combination of the first baseband stream and the second baseband stream, or a one of the first baseband stream and the second baseband stream, and is determined based on respective first and second received signal strength indicator or on respective first and second baseband stream distortion associated with the respective first and second baseband stream.

2. A hearing device as claimed in claim 1, wherein the second near field communication device is configured to transmit the preferred baseband stream, and the first near field communication unit is configured to receive the preferred baseband stream.

3. A hearing device as claimed in claim 1, wherein the second near field communication device is configured to transmit the second baseband stream, the first near field communication device is configured to receive the second baseband stream, and the first sub-unit is configured to determine a further preferred baseband stream from at least one of the first baseband stream and the second baseband stream.

4. A hearing device as claimed in any preceding claim, wherein at least one of the first receiver and the second receiver comprises a transceiver.

5. A hearing device as claimed in claim 3, wherein the further preferred baseband stream is determined based on respective first and second received signal strength indicator or on respective first and second baseband stream distortion associated with the respective first and second baseband stream.

6. A hearing device as claimed in any preceding claim, wherein the first and second near field communication devices are configured to operate within a bandwidth which is within the range of 3- 16 MHz.

7. A hearing device as claimed in any preceding claim, wherein the first and second receivers are each configured to receive an electromagnetic signal within one of a group of frequency ranges consisting of 400MHz - 6 GHz and 2.40-2.48 GHz.

8. A hearing device as claimed in any preceding claim, being one of the group consisting of: a hearing aid; a pair of headphones; a pair of earpieces, and a pair of implants.

9. A method of operating a hearing device comprising a first sub-unit having a first receiver and a second sub-unit having a second receiver, the method comprising:
receiving (310) a first signal from an electromagnetic field and deriving (320) a first baseband stream therefrom;
receiving (315) a second signal from the electromagnetic field and deriving (325) a second baseband stream therefrom;
transmitting, by a first near field communication device, the first baseband stream from the first sub-unit to the second sub-unit,
the second sub-unit further comprises configured to receiving, at the second sub-unit, the first baseband stream, by a second near field communication device,
**characterized in** determining a preferred baseband stream from at least one of the first baseband stream and the second baseband stream,
wherein determining a preferred baseband stream from at least one of the first baseband stream and the second baseband stream comprises making a determination based on respective first and second received signal strength indicator or on respective first and second baseband stream distortion associated with the respective first and second baseband stream;
wherein the preferred baseband stream is a result of a combination of the first baseband stream and the second baseband stream, or a one of the first baseband stream and the second baseband stream.

10. The method of claim 9 further comprising:
transmitting the preferred baseband stream by the second near field communication unit,
and receiving the preferred baseband stream by the first near field communication unit by.

11. The method of claim 9 further comprising:
transmitting (335) the second baseband stream by the second near field communication device, is configured to receiving the second baseband stream by the first near field communication device,
and determining a further preferred baseband stream from at least one of the first baseband stream and the second baseband stream by the first sub-unit.

## Patentansprüche

1. Ein Hörgerät, welches eine erste Untereinheit und eine zweite Untereinheit aufweist, wobei
die erste Untereinheit (10) einen ersten Empfänger (12) aufweist, welcher konfiguriert ist, um ein erstes Signal von einem elektromagnetischen Feld zu empfangen und davon einen ersten Basisband Stream abzuleiten; wobei
die zweite Untereinheit (20) einen zweiten Empfänger (22) aufweist, welcher konfiguriert ist, um ein zweites Signal von dem elektromagnetischen Feld zu empfangen und davon einen zweiten Basisband Stream abzuleiten;
wobei die erste Untereinheit ferner ein erstes Nahfeld Kommunikationsgerät (610) aufweist, welches konfiguriert ist, um den ersten Basisband Stream zu senden, wobei die zweite Untereinheit ferner ein zweites Nahfeld Kommunikationsgerät (615) aufweist, welches konfiguriert ist, um den ersten Basisband Stream zu empfangen,
**dadurch gekennzeichnet, dass**
die zweite Untereinheit konfiguriert ist, um einen bevorzugten Basisband Stream von zumindest einem von dem ersten Basisband Stream und dem zweiten Basisband Stream zu bestimmen;
wobei der bevorzugte Basisband Stream ein Ergebnis von einer Kombination von dem ersten Basisband Stream und dem zweiten Basisband Stream ist,
oder einer von dem ersten Basisband Stream und dem zweiten Basisband Stream ist und basierend auf einem entsprechenden ersten und zweiten empfangenen Signalstärkenindikator oder einer entsprechenden ersten und zweiten Basisband Stream Verzerrung bestimmt wird, welche mit dem entsprechenden ersten und zweiten Basisband Stream verknüpft ist.

2. Das Hörgerät gemäß Anspruch 1, wobei das zweite Nahfeld Kommunikationsgerät konfiguriert ist, um den bevorzugten Basisband Stream zu senden, und die erste Nahfeld Kommunikationseinheit konfiguriert ist, um den bevorzugten Basisband Stream zu empfangen.

3. Das Hörgerät gemäß Anspruch 1, wobei das zweite Nahfeld Kommunikationsgerät konfiguriert ist, um den zweiten Basisband Stream zu senden, wobei das erste Nahfeld Kommunikationsgerät konfiguriert ist, um den zweiten Basisband Stream zu empfangen, und die erste Untereinheit konfiguriert ist, um einen weiteren bevorzugten Basisband Stream von zumindest einem von dem ersten Basisband Stream und dem zweiten Basisband Stream zu bestimmen.

4. Das Hörgerät gemäß irgendeinem vorangehenden Anspruch, wobei zumindest einer von dem ersten Empfänger und dem zweiten Empfänger einen Transceiver aufweist.

5. Das Hörgerät gemäß Anspruch 3, wobei der weitere bevorzugte Basisband Stream basierend auf dem entsprechenden ersten und zweiten empfangenen Signalstärkenindikator oder der entsprechenden ersten und zweiten Basisband Stream Verzerrung bestimmt wird, welche mit dem entsprechenden ersten und zweiten Basisband Stream verknüpft ist.

6. Das Hörgerät gemäß irgendeinem vorangehenden Anspruch, wobei das erste und zweite Nahfeld Kommunikationsgerät konfiguriert sind, um innerhalb einer Bandbreite zu arbeiten, welche innerhalb des Bereichs von 3 - 16 MHz liegt.

7. Das Hörgerät gemäß irgendeinem vorangehenden Anspruch, wobei jeder von dem ersten und zweiten Empfänger konfiguriert ist, um ein elektromagnetisches Signal zu empfangen, welches innerhalb einer Gruppe von Frequenzbereichen liegt, welche aus 400 MHz - 6 GHz und 2.40 - 2.48 GHz besteht.

8. Das Hörgerät gemäß irgendeinem vorangehenden Anspruch, welches eines aus der Gruppe ist, welche aus einer Hörhilfe, ein Paar von Kopfhörern, ein Paar von Ohrmuscheln und ein Paar von Implantaten besteht.

9. Ein Verfahren zum Betreiben eines Hörgeräts, welches eine erste Untereinheit aufweist, die einen ersten Empfänger hat, und welches eine zweite Untereinheit aufweist, die einen zweiten Empfänger hat, wobei das Verfahren aufweist:
Empfangen (310) eines ersten Signals von einem elektromagnetischen Feld und
Ableiten (320) eines ersten Basisband Streams davon;
Empfangen (315) eines zweiten Signals von dem elektromagnetischen Feld und
Ableiten (325) eines zweiten Basisband Streams davon;
Senden des ersten Basisband Streams von der ersten Untereinheit zu der zweiten Untereinheit mittels eines ersten Nahfeld Kommunikationsgeräts,
wobei die zweite Untereinheit ferner aufweist konfiguriert zu sein zum
Empfangen des ersten Basisband Streams an der zweiten Untereinheit mittels eines zweiten Nahfeld Kommunikationsgeräts,
**gekennzeichnet durch**
Bestimmen eines bevorzugten Basisband Streams von zumindest einem von dem ersten Basisband Stream und dem zweiten Basisband Stream,
wobei das Bestimmen eines bevorzugten Basisband Streams von zumindest einem von dem ersten Basisband Stream und dem zweiten Basisband Stream aufweist
Vornehmen einer Bestimmung basierend auf einem entsprechenden ersten und zweiten empfangenen Signalstärkenindikator oder einer entsprechenden ersten und zweiten Basisband Stream Verzerrung, welche mit dem entsprechenden Basisband Stream verknüpft ist;
wobei der bevorzugte Basisband Stream ein Ergebnis von einer Kombination von dem ersten Basisband Stream und dem zweiten Basisband Stream ist,
oder einer von dem ersten Basisband Stream und dem zweiten Basisband Stream.

10. Das Verfahren gemäß Anspruch 9, welches ferner aufweist:
Senden des bevorzugten Basisband Streams mittels der zweiten Nahfeld Kommunikationseinheit, und
Empfangen des bevorzugten Basisband Streams mittels der ersten Nahfeld Kommunikationseinheit.

11. Das Verfahren gemäß Anspruch 9, welches ferner aufweist:
Senden (335) des zweiten Basisband Streams mittels des zweiten Nahfeld Kommunikationsgeräts ist konfiguriert, um den zweiten Basisband Stream mittels des ersten Nahfeld Kommunikationsgeräts zu empfangen, und
Bestimmen eines weiteren bevorzugten Basisband Streams von zumindest einem von dem ersten Basisband Stream und dem zweiten Basisband Stream mittels der ersten Untereinheit.

## Revendications

1. Dispositif auditif comprenant une première sous-unité et une deuxième sous-unité :
la première sous-unité (10) comprenant un premier récepteur (12) configuré pour recevoir un premier signal à partir d'un champ électromagnétique et en déduire un premier flux en bande de base ;
la deuxième sous-unité (20) comprenant un deuxième récepteur (22) configuré pour recevoir un deuxième signal à partir du champ électromagnétique et en déduire un deuxième flux en bande de base ;
dans lequel la première sous-unité comprend en outre un premier dispositif de communication en champ proche (610) configuré pour émettre le premier flux en bande de base, la deuxième sous-unité comprend en outre un deuxième dispositif de communication en champ proche (615) configuré pour recevoir le premier flux en bande de base, **caractérisé par le fait que** la deuxième sous-unité est configurée pour déterminer un flux en bande de base préféré parmi au moins le premier flux en bande de base et le deuxième flux en bande de base ;
dans lequel le flux en bande de base préféré est un résultat d'une combinaison du premier flux en bande de base et du deuxième flux en bande de base, ou un flux parmi le premier flux en bande de base, et le deuxième flux en bande de base, et est déterminé sur la base des indicateurs de puissance du premier et du deuxième signal reçu respectifs ou sur la base de la distorsion du premier et du deuxième flux en bande de base respectifs associés au premier et au deuxième flux en bande de base respectifs.

2. Dispositif auditif selon la revendication 1, dans lequel le deuxième dispositif de communication en champ proche est configuré pour émettre le flux en bande de base préféré, et dans lequel le premier dispositif de communication en champ proche est configuré pour recevoir le flux en bande de base préféré.

3. Dispositif auditif selon la revendication 1, dans lequel le deuxième dispositif de communication en champ proche est configuré pour émettre le deuxième flux en bande de base, le premier dispositif de communication en champ proche est configuré pour recevoir le deuxième flux en bande de base, et dans lequel la première sous-unité est configurée pour déterminer un autre flux en bande de base préféré à partir d'au moins un flux parmi le premier flux en bande de base et le deuxième flux en bande de base.

4. Dispositif auditif selon l'une quelconque des revendications précédentes, dans lequel au moins un parmi le premier récepteur et le deuxième récepteur comprend un émetteur-récepteur.

5. Dispositif auditif selon la revendication 3, dans lequel l'autre flux en bande de base préféré est déterminé sur la base des indicateurs de puissance du premier et du deuxième signal reçu respectifs ou de la distorsion du premier et du deuxième flux en bande de base respectifs associés au premier et au deuxième flux en bande de base respectifs.

6. Dispositif auditif selon l'une quelconque des revendications précédentes, dans lequel le premier et le deuxième dispositifs de communication en champ proche sont configurés pour fonctionner dans une largeur de bande qui est dans la plage de 3 à 16 MHz.

7. Dispositif auditif selon l'une quelconque des revendications précédentes, dans lequel le premier et le deuxième récepteurs sont configurés chacun pour recevoir un signal électromagnétique dans un groupe de plages de fréquences constituées des fréquences de 400 MHz à 6 GHz et de 2,40 à 2, 48 GHz.

8. Dispositif auditif selon l'une quelconque des revendications précédentes, parmi le groupe constitué de : une prothèse auditive ; une paire d'écouteurs ; une paire d'écouteurs intra-auriculaires, et une paire d'implants.

9. Procédé pour faire fonctionner un dispositif auditif comprenant une première sous-unité ayant un premier récepteur et une deuxième sous-unité ayant un deuxième récepteur, le procédé comprenant :
recevoir (310) un premier signal à partir d'un champ électromagnétique et dériver (320), à partir de celui-ci, un premier flux en bande de base ;
recevoir (315) un deuxième signal à partir du champ électromagnétique et dériver (325), à partir de celui-ci, un deuxième flux en bande de base ;
émettre, par un premier dispositif de communication en champ proche, le premier flux en bande de base de la première sous-unité à la deuxième sous-unité,
la deuxième sous-unité étant en outre configurée pour
recevoir, au niveau de la deuxième sous-unité, le premier flux en bande de base, par un deuxième dispositif de communication en champ proche,
**caractérisé par** : déterminer un flux en bande de base préféré parmi au moins le premier flux en bande de base et le deuxième flux en bande de base,
dans lequel la détermination d'un flux en bande de base préféré parmi au moins le premier flux en bande de base et le deuxième flux en bande de base comprend le fait d'effectuer une détermination sur la base des indicateurs de puissance du premier et
du deuxième signal reçu respectifs ou sur la base de la distorsion du premier et du deuxième flux en bande de base correspondants associés au premier et au deuxième flux en bande de base respectifs ;
dans lequel le flux en bande de base préféré est un résultat d'une combinaison du premier flux en bande de base et du deuxième flux en bande de base, ou un flux parmi le premier flux en bande de base et le deuxième flux en bande de base.

10. Procédé selon la revendication 9 comprenant en outre :
émettre le flux en bande de base préféré par la deuxième unité de communication en champ proche,
et recevoir le flux en bande de base préféré par la première unité de communication en champ proche.

11. Procédé selon la revendication 9 comprenant en outre :
émettre (335) le deuxième flux en bande de base par le deuxième dispositif de communication en champ proche, recevoir le deuxième flux en bande de base par le premier dispositif de communication en champ proche,
déterminer, par la première sous-unité, un autre flux en bande de base préféré à partir d'au moins un flux parmi le premier flux en bande de base et le deuxième flux en bande de base.
